(19) Europäisches Patentamt

European Patent Office

Office européen des brevets

(11) **EP 3 710 816 B1**

(12) **FASCICULE DE BREVET EUROPEEN**

(45) Date de publication et mention
de la délivrance du brevet:
**15.07.2026 Bulletin 2026/29**

(21) Numéro de dépôt: **18836282.6**

(22) Date de dépôt: **18.12.2018**

(51) Classification Internationale des Brevets (IPC):
**G01N 25/18** *(2006.01)*

(52) Classification Coopérative des Brevets (CPC):
**G01N 25/18**

(86) Numéro de dépôt international:
**PCT/FR2018/053356**

(87) Numéro de publication internationale:
**WO 2019/122672 (27.06.2019 Gazette 2019/26)**

(54) **PROCEDE DE CARACTERISATION ET DE CONTROLE DE L'HOMOGENEITE DE PIECES METALLIQUES FABRIQUEES PAR FRITTAGE LASER**

VERFAHREN ZUR CHARAKTERISIERUNG UND ÜBERWACHUNG DER HOMOGENITÄT VON DURCH LASERSINTERN HERGESTELLTEN METALLTEILEN

METHOD FOR CHARACTERIZING AND MONITORING THE HOMOGENEITY OF METAL PARTS MANUFACTURED BY LASER SINTERING

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.12.2017 FR 1762519**

(43) Date de publication de la demande:
**23.09.2020 Bulletin 2020/39**

(73) Titulaire: **Commissariat à l'Energie Atomique et aux Energies Alternatives
75015 Paris (FR)**

(72) Inventeurs:
• **MASKROT, Hicham**
**91310 MONTLHERY (FR)**
• **SEMEROK, Alexandre**
**91120 PALAISEAU (FR)**

(74) Mandataire: **Santarelli
Tour Trinity
1 bis Place de la Défense
92400 Courbevoie (FR)**

(56) Documents cités:
**FR-A1- 3 007 523**

• **ALEXANDRE SEMEROK ET AL: "Lock-in thermography for characterization of nuclear materials", EPJ NUCLEAR SCIENCES & TECHNOLOGIES, vol. 2, 1 January 2016 (2016-01-01), pages 20, XP055506820, DOI: 10.1051/epjn/2016015**
• **ZALDIVAR ESCOLA FACUNDO ET AL: "Characterization of Sintered Mixed Oxides by Photothermal Microscopy", INTERNATIONAL JOURNAL OF THERMOPHYSICS SPRINGER NEW YORK USA, SPRINGER NEW YORK LLC, US, vol. 37, no. 2, 11 January 2016 (2016-01-11), pages 1 - 18, XP035911048, ISSN: 0195-928X, [retrieved on 20160111], DOI: 10.1007/ S10765-015-2027-8**
• **TOLEV J ET AL: "Laser photothermal non-destructive inspection method for hairline crack detection in unsintered automotive parts: A statistical approach", NDT & E INTERNATIONAL, BUTTERWORTH-HEINEMANN, OXFORD, GB, vol. 43, no. 4, 1 June 2010 (2010-06-01), pages 283 - 296, XP026999388, ISSN: 0963-8695, [retrieved on 20100306]**

**Description**

**DOMAINE TECHNIQUE**

L'invention se situe dans le domaine de la fabrication de pièces métalliques par frittage et concerne plus spécifiquement une méthode non-destructive de caractérisation et du contrôle de l'homogénéité de pièces métallique pendant sa fabrication.

L'invention concerne également un dispositif pour mettre en œuvre le procédé selon l'invention.

**ÉTAT DE LA TECHNIQUE ANTÉRIEURE**

Dans de nombreux secteurs industriels tels que le génie mécanique, l'électronique, l'aéronautique, la métallurgie, ou le contrôle non-destructif, la connaissance précise de l'homogénéité et des propriétés thermiques d'un matériau est une information précieuse. Ces informations sont obtenues dans le cadre d'un contrôle qualité effectués généralement après la fabrication des pièces.

On connait plusieurs techniques pour effectuer ce contrôle telles que par exemple la radiologie, le contrôle par ultrason, la radiométrie photothermique modulée.

L'examen de la structure ou de l'état interne d'un objet par radiographie consiste à faire traverser celui-ci par un rayonnement électromagnétique de très courte longueur d'onde (rayons X ou $\gamma$) et de recueillir les modulations d'intensité du faisceau sous forme d'une image sur un récepteur approprié ou, dans la plupart des cas, un film.

Selon le même principe, on peut obtenir des images en utilisant d'autres particules que les photons, et ainsi, mettre en œuvre des techniques comme la neutronographie.

La radiographie est une méthode de contrôle non destructif qui consiste à obtenir une image de la densité de matière d'un objet traversé par un rayonnement électromagnétique X ou gamma. Le principe de la méthode est basé sur l'absorption différentielle du milieu en fonction du numéro atomique des atomes le composant et de la masse volumique. Tout manque de matière induira une plus faible absorption et donc, localement, une plus forte densité optique sur le film ou un niveau de gris plus élevé dans le cas des images numériques. En radiologie industrielle, les rayons X sont produits, le plus souvent par un tube à rayons X, ou par un accélérateur de particules pour les applications à haute énergie. Les sources de rayonnement gamma utilisées industriellement sont l'iridium 192, le cobalt 60 et le sélénium 75. La radiographie est une technique qui permet de visualiser les manques de matière du volume de l'objet contrôlé sur une image à deux dimensions.

La neutronographie est une technique de mesure nucléaire (contrôle non destructif) dont le principe est similaire à celui de la radiographie à rayons X mais qui emploie des neutrons comme source de rayonnement. Une image en transmission est produite en interposant l'objet inspecté entre une source neutronique (souvent issue d'un réacteur) et un système de détection de neutrons. L'atténuation du flux de neutrons est plus ou moins prononcée en fonction de la nature de la matière rencontrée, donnant alors des différences de contrastes permettant une analyse du contenu de l'objet.

Le principe du contrôle par ultrasons consiste à émettre et propager une onde ultrasonore dans la pièce à inspecter. Puis, on recueille et analyse l'onde à l'issue de son interaction avec le matériau. Sur la base de ce principe très général, il existe de nombreuses techniques spécifiques, suivant que le contrôle soit effectué en transmission ou bien en réflexion, ou bien que les dispositifs en émission et en réception soient confondus ou non, et enfin, suivant le type et l'inclinaison des ondes ultrasonores utilisées, etc. La modalité de contrôle la plus répandue, dite en réflexion, est comparable à l'échographie médicale. L'émetteur et le récepteur (confondus ou non) sont positionnés du même côté de la pièce. Le récepteur recueille les échos engendrés par réflexion ou diffraction sur les obstacles rencontrés par l'onde, tels que les défauts, les interfaces entre matériaux ou encore la surface de la pièce. Les dispositifs émetteurs et récepteurs, dits « traducteurs ultrasonores », sont en général basés sur l'effet piézo-électrique. L'élément principal, le transducteur, est constitué d'une pastille piézo-électrique convertissant un signal électrique en vibration mécanique et inversement.

On connait aussi des méthodes de détermination des paramètres thermophysiques d'un corps à partir de l'analyse des ondes thermiques émises par le corps. Ces méthodes se différencient par :

- le type de chauffage (temporel, spatial),
- l'emplacement de la source de chaleur et du détecteur sur l'objet de diagnostics,
- l'emplacement de la source de chaleur et du point de mesure, l'un par rapport à l'autre (au centre du faisceau ou à côté),
- le nombre de points de mesure. Il existe déjà nombre de méthodes théoriques pour différents types de caractérisation.

En associant la mesure de la température à la modélisation du chauffage, il est possible de déterminer, sous certaines conditions, les propriétés thermiques de la surface. Cette technique peut être employée plus spécifiquement, pour mesurer de manière non destructive et à distance, les propriétés d'une couche ou d'un revêtement sur un substrat connu. Dans le domaine de contrôle thermique actif non destructif, on peut distinguer quatre méthodes différentes énumérées et décrites, ci-après:

- Méthode impulsionnelle ;
- Méthode de chauffage continu ;
- Méthode impulsionnelle périodique ;
- Méthode par radiométrie photothermique modulée à détection synchrone, appelée aussi comme « *Lock-in* ».

Dans le cas du chauffage impulsionnel, le matériau est soumis à une seule impulsion issue de la source de chaleur (par exemple, un laser impulsionnel), de paramètres énergétiques connus. En utilisant la courbe de refroidissement du matériau, on obtient alors par résolution inverse de l'équation de transfert de la chaleur, les propriétés thermophysiques recherchées. Cette technique permet d'obtenir une solution à partir des données d'une mesure relativement courte.

Nombre des méthodes impulsionnelles ont été développées pour caractériser les matériaux homogènes et revêtus d'une ou plusieurs couches. La méthode la plus utilisée est une mesure d'un des phénomènes thermiques en face avant de l'échantillon, c'est-à-dire de la surface chauffée. La plupart des études sont consacrées à des mesures au centre du faisceau laser, mais on peut suivre aussi la variation de la température dans une direction latérale. Une des applications est la caractérisation des défauts. Les propriétés thermiques que l'on cherche à déterminer sont la diffusivité, l'effusivité, la conductivité thermique ou les combinaisons de ces propriétés.

La deuxième catégorie de méthodes de pyrométrie active est basée sur un chauffage continu de la surface. Comme pour la première méthode, le paramètre principal est la température de surface et son écart par rapport aux matériaux de référence appelé contraste thermique. L'avantage de cette méthode, comme dans le premier cas, est sa rapidité mais, à la différence de la faiblesse du signal de température de refroidissement du chauffage impulsionnel, le chauffage continu permet de faire des mesures avec des températures plus élevées donc plus précises. Cependant, chauffer conduit au risque de surchauffe de la matière et, en conséquence, de modifier ses propriétés.

Comme indiqué précédemment, le chauffage en échelon permet, en comparant le modèle estimé avec l'expérience, de déterminer les caractéristiques de la couche.

Le chauffage impulsionnel répétitif permet de palier les inconvénients des deux méthodes précédentes. La température est maintenue à un niveau suffisant pour les mesures, tout en réduisant le risque de surchauffe de la surface.

Tolev, Jordan & Mandelis, Andreas. (2010). "Laser photothermal non-destructive inspection method for hairline crack detection in unsintered automotive parts: A statistical approach." NDT & E International. 43. 283-296. divulgue l'inspection photo thermique des pieces vertes. Un autre exemple de caractérisation photothermique d'une pièce métallique est divulgué par FR 3 007 523 A1.

Un problème des méthodes ci-dessus provient du fait qu'elles ne peuvent pas être mise en œuvre pendant la fabrication de la pièce car l'émissivité du matériau n'est généralement pas connue, conduisant ainsi à des résultats imprécis, voir des mesures incorrectes de la température. De plus, pour déterminer les propriétés thermiques à partir des mesures de la température, il faut connaitre le flux de chauffage absorbé par la surface et, donc, la puissance laser ainsi que le coefficient d'absorption.

Le but de l'invention est de réaliser une détection en temps réel de défaut sur une pièce fabriquée par frittage sans connaitre à priori le flux de chauffage absorbé par la surface et, donc, la puissance laser ainsi que le coefficient d'absorption.

## EXPOSÉ DE L'INVENTION

Ce but est atteint au moyen d'un procédé non-destructif de caractérisation et de contrôle de l'homogénéité de pièces métallique comportant plusieurs zones distinctes fabriquées par frittage, dans lequel on applique un rayonnement laser successivement sur chaque zone et on effectue simultanément, en temps réel, une analyse de chaque zone frittée par radiométrie active laser à détection synchrone.

Préférentiellement, on applique le rayonnement laser successivement sur des sous-zones successives de chaque zone de manière à utiliser un rayonnement laser de faible puissance, la taille desdites sous-zones présentant une forme régulière sur laquelle le rayonnement laser est appliqué ligne par ligne de manière à former en temps réel une image de la zone frittée.

Selon une autre caractéristique de l'invention, on applique sur chaque zone un chauffage modulé en fréquence générant à chaque fréquence une seule onde thermique, et pour chaque fréquence, on mesure en temps réel le déphasage entre le signal lumineux du laser appliqué à la pièce et le signal thermique émis par la pièce.

Selon l'invention, l'épaisseur $L(\mu m)$ et la diffusivité thermique D ($m^2/s$) de chaque zone sont déterminées par les formules suivantes :

$$L = \frac{r_0}{\zeta_\varphi} \ln \frac{90}{|\varphi_{min}|} \qquad (1)$$

$$D = \frac{1}{\zeta_f} f_{min} L r_0 \qquad (2)$$

où $r_0$ est le rayon de faisceau laser à 1/e en intensité;

$\varphi_{min}$ et $f_{min}$ représentent respectivement le déphasage minimum et la cadence faisceau laser;

$\zeta_\varphi$ et $\zeta_f$ représentent des coefficients connus qui dépendent respectivement du déphasage minimum $\varphi_{min}$ et du rapport $r_0/L$.

Le procédé selon l'invention est mis en œuvre par un dispositif comportant une source laser adaptée pour appliquer successivement sur chaque zone de la pièce contrôlée un rayonnement laser, un détecteur de rayonnement infrarouge adapté pour capter et mesurer en temps réel le rayonnement thermique émis par chaque zone de ladite pièce, un détecteur synchrone destiné détecter le déphasage entre le signal lumineux du rayonnement laser et le signal thermique du rayonnement infrarouge émis par chaque zone de ladite pièce.

La source laser du dispositif selon l'invention est configurée pour appliquer sur chaque zone un chauffage modulé générant à chaque fréquence une seule onde thermique, et le détecteur synchrone est configuré pour mesurer, pour chaque fréquence, le déphasage entre le signal lumineux du laser appliqué à la pièce et le signal thermique émis par la pièce.

## BRÈVE DESCRIPTION DES DESSINS

D'autres caractéristiques et avantages de l'invention ressortiront de la description qui va suivre, prise à titre d'exemple non limitatif, en référence aux figures annexées dans lesquelles :

- la figure 1 illustre schématiquement un échantillon d'une pièce métallique obtenus par la méthode de la fabrication additive en utilisant le procédé selon l'invention ;
- la figure 2 illustre les dimensions des différentes zones de l'échantillon de la figure 1 ;
- la figure 3 illustre schématiquement un dispositif de caractérisation et de contrôle de l'homogénéité de pièces métallique selon l'invention ;
- la figure 4 illustre schématiquement un mode de réalisation du système optique utilisé dans le dispositif de caractérisation et de contrôle de l'homogénéité de pièces métallique selon l'invention ;
- la figure 5 est une courbe illustrant les variations des déphasages mesurés en fonction de la fréquence pour l'échantillon de la figure 1 pour deux épaisseurs différentes ;
- la figure 6 illustre schématiquement un premier montage pour mesurer le déphasage en fonction de la fréquence pour la pièce métallique de la figure 4 en direction de l'axe x ;
- la figure 7 est une courbe illustrant les déphasages mesuré par le montage de la figure 6 ;
- la figure 8 illustre schématiquement un deuxième montage pour mesurer le déphasage en fonction de la fréquence pour la pièce métallique de la figure 4 en direction de l'axe z ;
- la figure 9 est une courbe illustrant les déphasages mesuré par le montage de la figure 8.

## EXPOSÉ DÉTAILLÉ DE MODES DE RÉALISATION PARTICULIERS

L'invention sera décrite dans une application pour la fabrication et la caractérisation en temps réel de pièces métalliques utilisées dans l'industrie nucléaire.

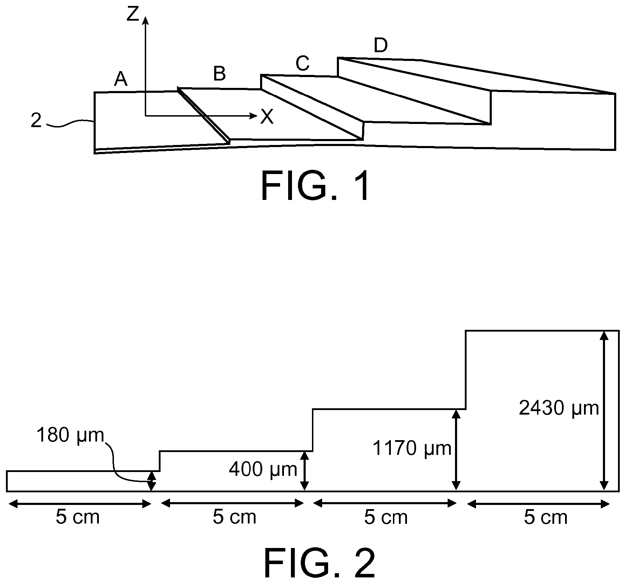

A titre d'exemple illustratif, la méthode sera appliquée sur un échantillon métallique 2 de dimensions 20 mm × 20 mm (latérales) et 180 μm à 2430 μm d'épaisseurs obtenus par la méthode de la fabrication additive en utilisant de la poudre d'Inox 316 L illustrées par les figures 1 et 2.

L'échantillon 2 comporte quatre zones distinctes A, B, C et D ayant respectivement pour épaisseurs 180 ± 20 μm (zone A), 400 ± 20 μm (zone B), 1170 ± 20 μm (zone C), 2430 ± 20 μm (zone D). La rugosité surfacique est de ± 20 μm. La diffusivité thermique de cet échantillon en Inox 316 L est D = 0,039 ± 0,004 cm$^2$/s [4] pour la température ambiante.

Les zones A et B ont été examinées par le procédé selon l'invention au cours de la fabrication au moyen du dispositif 3. Ce dernier comporte une source laser à fibre 4 de longueur d'onde 1080 nm pour chauffer l'échantillon 2 avec une puissance moyenne accordable de 0 à 50 W. Cette puissance moyenne peut être modulée. Le rayon $r_O$ du faisceau collimaté au niveau de l'échantillon 2 est égal à 1740 μm (rayon à 1/e en intensité). La puissance laser (amplitude et cadence) est gérée par un générateur de fonction 6. La sortie du générateur 6 (impédance de sortie : 50 Ω) est séparée en deux voies, une pour le fonctionnement du laser (impédance d'entrée : 50 Ω) et l'autre pour l'utilisation comme le signal de référence d'un amplificateur à détection synchrone (impédance d'entrée: 1 MΩ).

Le générateur de fonction 6 peut produire un signal continu ou un signal modulé sinusoïdal, rectangulaire ou triangulaire, d'amplitude et de fréquence variable. La source laser 4 délivre jusqu'à 50 W de puissance de sortie en continue et jusqu'à 25 W (puissance moyenne) en sinusoïdal, avec une qualité de faisceau qui est proche de la limite de diffraction avec $M^2 < 1,1$. Les caractéristiques du laser sont présentées dans le ci-dessous.

**Caractéristiques de la source laser à fibre 4.**

| Mode d'opération | | Continu, modulé |
|---|---|---|
| Longueur d'onde centrale | nm | 1080 |
| Forme de faisceau | | Gaussien, collimaté |
| Qualité de faisceau ($M^2$) | | 1,1 |
| Diamètre du faisceau à 1/e | μm | 1740 ± 30 |
| Puissance maximale (crête ou cw) | W | 50 |
| Cadence de modulation | Hz | ≤ 5 kHz |
| Tension maximale du générateur | V | 5 |
| Dimensions | mm | 448 x 451 x 132 |

La source laser 4 dispose d'un faisceau infrarouge de faible puissance pour indiquer la direction du faisceau et pour faciliter le réglage optique des échantillons avant les essais expérimentaux. La puissance moyenne du signal laser en régime sinusoïdal dépend faiblement de la cadence appliquée. La variation de la puissance du signal laser reste faible, de sorte qu'on peut considérer que la température moyenne de chauffage reste la même pour les cadences de 1 Hz à 1 kHz. Cela permet de conserver les mêmes propriétés thermiques pendant toute la durée des mesures avec les cadences différentes, ce qui assure une meilleure précision des mesures.

Le dispositif 3 comporte en outre un détecteur IR 10 pour mesurer le rayonnement thermique de l'échantillon 2 lorsque ce dernier est éclairé par la source laser 4. La plage spectrale utile du détecteur IR 10 s'étend de 1,5 μm à 11 μm. Une lentille convexe 12 en ZnSe est utilisée pour imager la zone chauffée retenue sur une surface du détecteur de rayon 800 μm. Un filtre en germanium 14, transmettant seulement les longueurs d'onde comprises entre 2 μm et 14 μm, a été placé devant le détecteur IR 10, afin d'éviter que la réflexion diffuse du signal laser ne perturbe la détection (car le détecteur a une sensibilité résiduelle à la longueur d'onde 1 μm).

Un amplificateur à détection synchrone 16 est utilisé pour déterminer le déphasage entre le signal lumineux du laser et le signal thermique en fonction de la fréquence du signal laser.

L'amplificateur à détection synchrone 16 est relié à un oscilloscope électronique 17. La puissance du signal laser est contrôlée par un détecteur de puissance laser 18 reliée également à l'oscilloscope électronique 17.

L'analyse de la courbe de déphasage permet de déterminer certaines propriétés de l'échantillon 2.

Pour éviter les retours de faisceau dans le laser, la surface de l'échantillon est placée avec un faible angle θ par rapport au plan perpendiculaire à l'axe normal incident. La taille du faisceau est alors multipliée par cos θ dans une direction. On choisit θ faible c'est-à-dire inférieur à 10° de façon à être dans le cadre de l'approximation cos θ ~ 1.

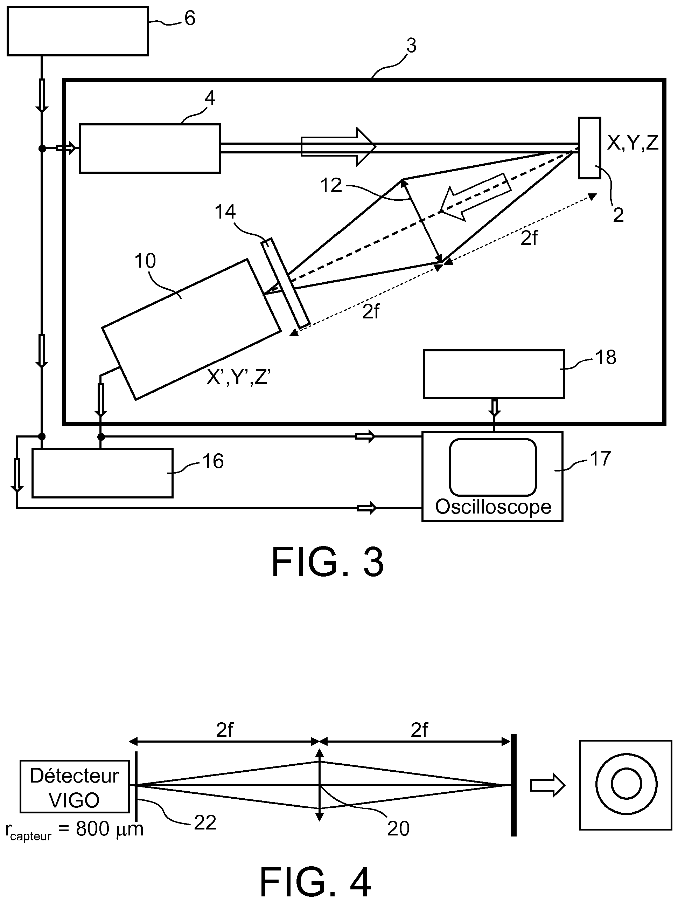

La figure 4 représente schématiquement le système optique utilisé dans le dispositif 1. Ce système optique comporte une lentille ZnSe 20 (domaine spectral de transmission de 0,6 μm à 15 μm) utilisée pour focaliser le flux thermique sur la zone sensible du détecteur IR 10 et un filtre Germanium 22 (domaine spectral de transmission de 1,8 μm à 23 μm) pour couper toutes les longueurs d'onde jusqu'à 1,8 μm, filtrant ainsi la longueur d'onde du laser.

La lentille convexe ZnSe 20, de distance focale 50 mm et de diamètre 25 mm, fait image sur le détecteur IR 10 le centre de la zone chauffée par le laser. La surface de capture du détecteur IR a pour rayon 800 μm. Pour n'imager que la partie centrale de la zone chauffée par le faisceau laser limitée à la moitié de son rayon, soit $r_{chauffage}/2 = 870$ μm, le grandissement $r_{capteur}/r_{collecté}$ est supérieur ou égal à 1 et la distance lentille échantillon est de 2f = 100 mm.

A titre d'exemple de mise en œuvre du procédé selon l'invention, les courbes de déphasage en fonction de la fréquence du signal laser seront déterminées pour les zones A et B de l'échantillon 2. Les courbes de déphasage obtenus permettent de déterminer la cadence optimale pour une meilleure distinction (Δφ maximal) entre les zones A et B. Toutes les mesures ont été réalisées du côté homogène où les paliers de l'échantillon ne sont pas visibles.

Une fois la cadence optimale déterminée, le faisceau laser est déplacé suivant l'axe x et l'axe z pour étudier l'homogénéité des propriétés de l'échantillon selon ces axes. Les axes ont été choisis de telle sorte qu'il soit possible de changer l'épaisseur suivant l'axe x sans changement d'épaisseur suivant l'axe z.

En fonctionnement, une zone est chauffée avec la source Laser 4, en variant la fréquence du signal laser de 1 à 200 Hz. La puissance moyenne est de 11 W (amplitude du générateur 3 V). Sachant que la variation de la fréquence affecte très peu la puissance en sortie du laser, la variation de la température doit être minime afin de ne pas affecter les mesures avec les cadences différentes.

Les mesures ont été effectuées sur deux zones d'épaisseurs différentes, l'une à 180 $\mu$m et l'autre à 400 $\mu$m.

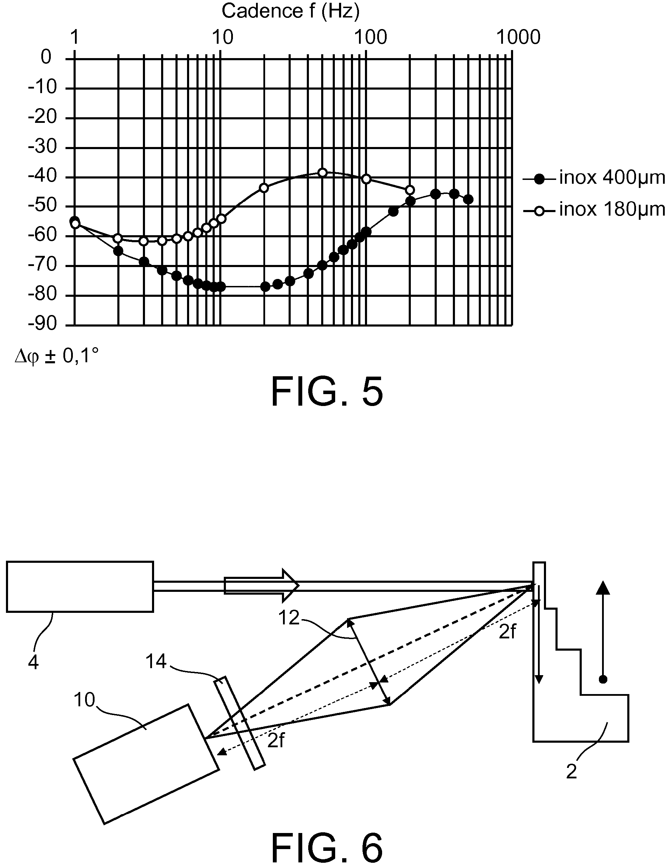

La figure 5 représente les déphasages obtenus. Cette courbe permet de déterminer le déphasage minimum $\varphi_{min}$ et la fréquence correspondante $f_{min}$ et, par les formules suivantes (1) et (2), l'épaisseur $L$ ($\mu$m) et la diffusivité thermique $D$ (m$^2$/s) dans les zones testées:

$$L = \frac{r_0}{\zeta_\varphi} \, ln \, \frac{90}{|\varphi_{min}|} \tag{1}$$

$$D = \frac{1}{\zeta_f} f_{min} \, L \, r_0 \tag{2}$$

où $r_0$ est le rayon du faisceau laser à 1/e en intensité; $\zeta_\varphi$ et $\zeta_f$ sont des coefficients connus qui dépendent respectivement du $\varphi_{min}$ et du rapport $r_0/L$.

Les résultats de mesure sont présentés dans le tableau 1 suivant :

**Résultats de mesures avec échantillon 2.**

|  | $\varphi_{min}$ | $f_{min}$ |
|---|---|---|
| Epaisseur $L$ = 180 $\mu$m | -77,1 $\pm$ 0,1° | 9 Hz |
| Epaisseur $L$ = 400 $\mu$m | -61,7 $\pm$ 0,1° | 3 Hz |

A partir de ces résultats et les coefficients $\zeta_\varphi$ et $\zeta_f$ présentés ci-dessous, il est possible de déterminer l'épaisseur et la diffusivité dans les zones A et B qui sont présentées dans le tableau ci-après.

$\zeta_\varphi$ = 1,535 $\pm$ 0,005 (180 $\mu$m) et 1,53 $\pm$ 0,005 (400 $\mu$m)
$\zeta_f$ = 0,534 $\pm$ 0,005 (180 $\mu$m) et 0,472 $\pm$ 0,005 (400 $\mu$m)

Les épaisseurs et les diffusivités thermiques déterminées par la méthode par radiométrie photothermique modulée pour l'échantillon 2 sont données par le tableau 2 suivant :

|  | Mesuré par Lock-in | Référence pour T = 400 K |
|---|---|---|
| Epaisseur $L$ (zone A) | 175,3 $\pm$ 26 $\mu$m | 180 $\pm$ 20 $\mu$m |
| Epaisseur $L$ (zone B) | 429,3 $\pm$ 30 $\mu$m | 400 $\pm$ 20 $\mu$m |
| $D$ en zone A | 0,051 $\pm$ 0,005 cm$^2$/s | 0,047 $\pm$ 0,003 cm$^2$/s |
| $D$ en zone B | 0,047 $\pm$ 0,005 cm$^2$/s | 0,047 $\pm$ 0,003 cm$^2$/s |

Ces épaisseurs déterminées par la méthode radiométrie modulée sont en bonne correspondance avec les épaisseurs mesurées par le pied à coulisse. Les diffusivités thermiques mesurées dans deux zones différentes de l'échantillon 2 sont les mêmes.

Les mesures effectuées permettent, dans un premier temps, d'analyser l'échantillon 2 dans la direction où il y a un changement d'épaisseur, et dans un deuxième temps, dans la direction où il n'y a pas de changement visible ni d'épaisseur, ni de propriétés thermophysiques.

A cet effet, la fréquence du rayon laser est fixe, et est égale à celle qui donne le plus grand écart de déphasage en passant d'une zone à l'autre c'est-à-dire à environ 20 Hz, ce qui permet de bien mettre en évidence les différents paliers.

La figure 6 illustre le premier cas d'analyse de l'échantillon 2 dans la direction où il y a un changement d'épaisseur.

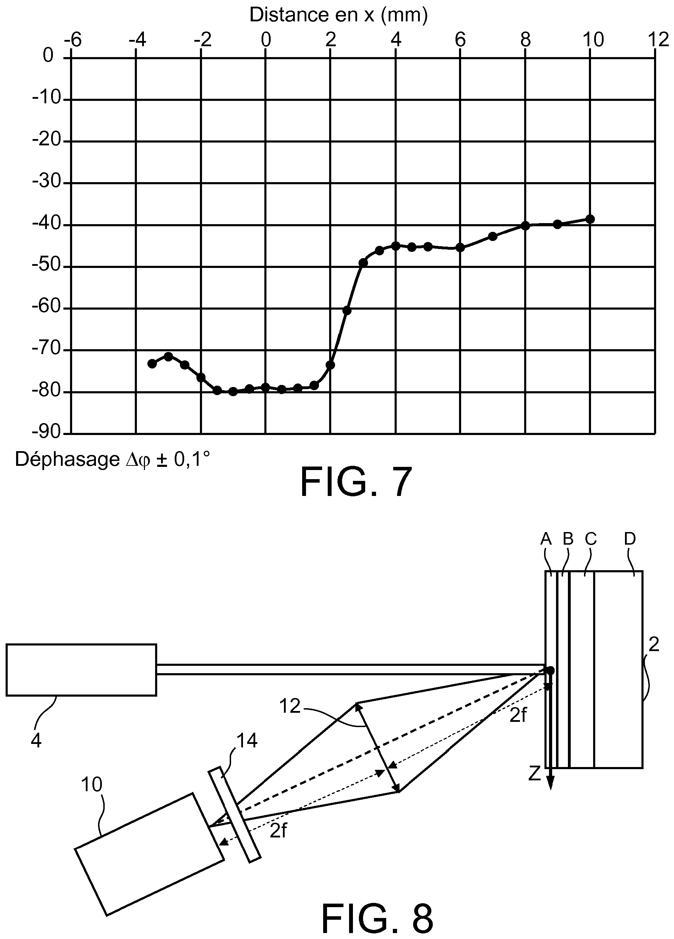

Dans ce cas, l'échantillon 2 est chauffé dans la zone A d'épaisseur 180 $\mu$m, puis est déplacé suivant l'axe x (vers la zone B) sur une distance de 15 mm avec un pas de 0,5 mm. Les déphasages mesurés pour chaque position sont présentés par la courbe de la figure 7.

Les deux paliers de déphasages représentent deux épaisseurs à deux paliers situés à -80° et à -45°. Un dernier palier est situé à -40°. La fréquence choisie est de 20 Hz pour bien différencier les deux paliers.

On remarque que la fréquence n'est pas adaptée pour montrer la différence entre les deux derniers paliers de 400 et 1170 $\mu$m (Figure 2).

La première partie de la courbe située pour x entre -4 et -2 mm montre un défaut dû à la fabrication de la pièce.

On observe que la méthode peut révéler facilement la non homogénéité (un palier) d'une pièce contrôlée. La résolution spatiale est d'environ un millimètre et elle est déterminée par la largeur de la zone testée par le détecteur VIGO (1,6 mm). Cette résolution peut être améliorée par l'utilisation d'un chemin optique avec une plus haute résolution.

La figure 8 illustre le deuxième cas d'analyse de l'échantillon 2 dans la direction où il n'y pas de changement d'épaisseur.

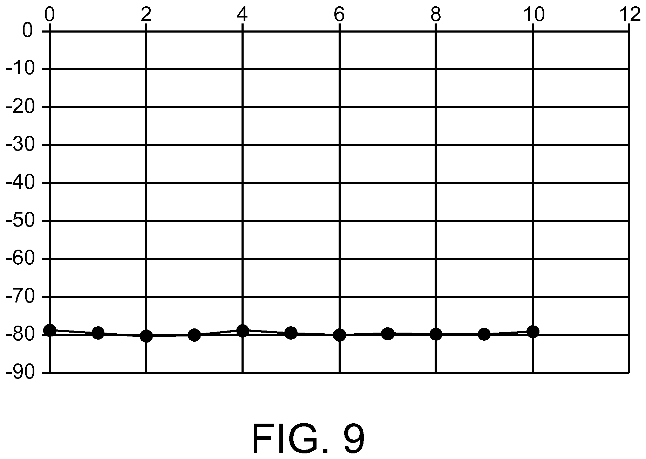

Dans ce cas, l'échantillon est chauffé dans la zone A d'épaisseur 180 $\mu$m, puis est déplacé suivant l'axe z sur une distance de 10 mm avec un pas de 0,5 mm. Les déphasages mesurés pour chaque position sont présentés par la courbe de la figure 9.

L'échantillon est chauffé dans la zone A d'épaisseur 180 $\mu$m, puis est déplacé suivant l'axe z sur une distance de 10 mm avec un pas de 0,5 mm.

On remarque une homogénéité du déphasage. En effet, la courbe est plane et est située à -80°. Ceci explique que la pièce ne présente aucun défaut interne ou externe.

Les mesures sont effectuées en temps réel, c'est-dire, au cours de la fabrication de la pièce. Ainsi, il est possible de corriger en temps réel le processus de fabrication des pièces par frittage.

**Revendications**

1. Procédé non-destructif de caractérisation et de contrôle de l'homogénéité d'une pièce métallique (2) fabriquée par frittage comportant plusieurs zones distinctes, le procédé étant **caractérisé en ce qu'**il comprend, pendant la fabrication de ladite pièce, les étapes suivantes :

   - on applique un rayonnement laser successivement sur chaque zone et on effectue simultanément en temps réel une analyse de chaque zone frittée par radiométrie active laser à détection synchrone,
   - on applique sur chaque zone un chauffage modulé en fréquence générant à chaque fréquence une seule onde thermique, et pour chaque fréquence, on mesure, en temps réel, le déphasage entre le signal lumineux du laser appliqué à la pièce et le signal thermique émis par la pièce,et,
   - on détermine l'épaisseur L ($\mu$m) et la diffusivité thermique D (m$^2$/s) de chaque zone par les formules suivantes :

$$L = \frac{r_0}{\zeta_\varphi} ln \frac{90}{|\varphi_{min}|} \qquad (1)$$

$$D = \frac{1}{\zeta_f} f_{min} L r_0 \qquad (2)$$

   où $r_0$ est le rayon de faisceau laser à 1/e en intensité ;
   $\varphi_{min}$ et $f_{min}$ représentent respectivement le déphasage minimum et la cadence faisceau laser ;
   $\zeta_\varphi$ et $\zeta_f$ représentent des coefficients connus qui dépendent respectivement du déphasage minimum $\varphi_{min}$ et du rapport $r_0/L$.

2. Procédé selon la revendication 1 dans lequel on applique le rayonnement laser successivement sur des sous-zones de chaque zone.

3. Procédé selon la revendication 2, dans lequel la taille desdites sous-zones présente une forme régulière sur laquelle le rayonnement laser est appliqué ligne par ligne de manière à former en temps réel une image de la zone frittée.

**Patentansprüche**

1. Zerstörungsfreies Verfahren zum Charakterisieren und Prüfen der Homogenität eines durch Sintern hergestellten Metallteils (2), das mehrere unterschiedliche Bereiche beinhaltet, wobei das Verfahren **dadurch gekennzeichnet ist, dass** es bei der Herstellung des Teils die folgenden Schritte umfasst:

   - Aufbringen einer Laserstrahlung nacheinander auf jeden Bereich und gleichzeitiges Durchführen in Echtzeit einer Analyse jedes gesinterten Bereichs durch aktive Laserradiometrie mit synchroner Erkennung,
   - Aufbringen einer frequenzmodulierten Heizung auf jeden Bereich, die bei jeder Frequenz eine einzige Wärmewelle erzeugt, und für jede Frequenz Messen in Echtzeit der Phasenverschiebung zwischen dem auf das Teil aufgebrachten Lichtsignal des Lasers und dem vom Teil emittierten Wärmesignal, und
   - Bestimmen der Dicke L ($\mu$m) und die Wärmeleitfähigkeit D (m$^2$/s) jedes Bereichs durch die folgenden Formeln:

$$L = \frac{r_0}{\zeta_\varphi} In \frac{90}{|\varphi_{min}|} \quad (1)$$

$$D = \frac{1}{\zeta_f} f_{min} L r_0 \, (2)$$

   wobei $r_0$ der Laserlichtstrahl bei 1/e der Intensität ist;
   $\varphi_{min}$ und $f_{min}$ jeweils die minimale Phasenverschiebung und den Laserstrahltakt darstellen;
   $\zeta_\phi$ und $\zeta_f$ bekannte Koeffizienten darstellen, die jeweils von der minimalen Phasenverschiebung $\varphi_{min}$ und dem Verhältnis $r_0/L$ abhängen.

2. Verfahren nach Anspruch 1, wobei die Laserstrahlung nacheinander auf Unterbereiche jedes Bereichs aufgebracht wird.

3. Verfahren nach Anspruch 2, wobei die Größe der Unterbereiche eine regelmäßige Form aufweist, auf welche die Laserstrahlung Zeile für Zeile aufgetragen wird, um in Echtzeit ein Bild des gesinterten Bereichs zu bilden.


**Claims**

1. A Non-destructive method for characterising and monitoring the homogeneity of a metal part (2) manufactured by sintering including several distinct zones, wherein, upon manufacturing said part, the method is **characterised by** the following steps:

   - a laser radiation is successively applied on each zone and an analysis of each sintered zone is simultaneously made in real time by synchronous detection active laser radiometry,
   - a frequency modulated heating generating at each frequency a single thermal wave is applied on each zone, and for each frequency, the phase shift between the luminous signal of the laser applied to the part and the thermal signal emitted by the part is measured in real time, and,
   - the thickness L ($\mu$m) and thermal diffusivity D (m$^2$/s) of each zone are determined by the following formulae:

$$L = \frac{r_0}{\zeta_\varphi} ln \frac{90}{|\varphi_{min}|} \qquad (1)$$

$$D = \frac{1}{\zeta_f} f_{min} L r_0 \qquad (2)$$

   where $r_0$ is the laser beam radius at 1/e intensity;
   $\varphi_{min}$ and $f_{min}$ represent the minimum phase shift and the laser beam rate respectively;
   $\zeta_\varphi$ and $\zeta_f$ represent known coefficients which depend on the minimum phase shift $\varphi_{min}$ and the ratio $r_0/L$ respectively.

2. The method according to claim 1, wherein the laser radiation is successively applied on sub-zones of each zone so as to use a low power laser radiation.

3. The method according to claim 2, wherein the size of said sub-zones has a regular shape on which the laser radiation is applied row by row so as to form an image of the sintered zone in real time.

# FIG. 1

# FIG. 2

FIG. 3

$r_{capteur} = 800\ \mu m$

FIG. 4

**FIG. 5**

**FIG. 6**

Distance en x (mm)

Déphasage Δφ ± 0,1°

# FIG. 7

# FIG. 8

FIG. 9

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

• FR 3007523 A1

**Littérature non-brevet citée dans la description**

• **TOLEV** ; **JORDAN** ; **MANDELIS** ; **ANDREAS**. Laser photothermal non-destructive inspection method for hairline crack detection in unsintered automotive parts: A statistical approach. *NDT & E International*, 2010, vol. 43, 283-296